Europäisches Patentamt

**European Patent Office**

**Office européen des brevets**

(11) Veröffentlichungsnummer: **0 086 358**
**A1**

(12) **EUROPÄISCHE PATENTANMELDUNG**

(21) Anmeldenummer: **83100612.7**

(22) Anmeldetag: **25.01.83**

(51) Int. Cl.³: **C 07 D 321/06, C 08 J 11/00, C 07 D 323/00**

(30) Priorität: **06.02.82 DE 3204078**

(43) Veröffentlichungstag der Anmeldung: **24.08.83**
**Patentblatt 83/34**

(84) Benannte Vertragsstaaten: **DE FR GB**

(71) Anmelder: **BAYER AG, Zentralbereich Patente, Marken und Lizenzen, D-5090 Leverkusen 1, Bayerwerk (DE)**

(72) Erfinder: **Krimm, Heinrich, Dr., Heyenbaumstrasse 65, D-4150 Krefeld (DE)**
Erfinder: **Buysch, Hans-Josef, Dr., Brandenburger Strasse 28, D-4150 Krefeld (DE)**

(54) **Verfahren zur Herstellung von Tetramethylencarbonat und Bis-tetramethylendicarbonat.**

(57) Tetramethylencarbonat und Bis-tetramethylendicarbonat werden in der Weise hergestellt, daß man aus dem Tetramethylenpolycarbonat die zu dessen Herstellung verwendeten Katalysatoren, deren Folgeprodukte und/oder sonstigen Fremdstoffe entfernt, das so vorbehandelte Polycarbonat auf zur Depolymerisation ausreichende Temperatur erhitzt und die entstehenden cyclischen Kohlensäureester durch Destillation unter vermindertem Druck abtrennt. Tetramethylencarbonat und Bis-tetramethylendicarbonat können zur Herstellung von hochmolekularen Polycarbonaten verwendet werden.

BAYER AKTIENGESELLSCHAFT          5090 Leverkusen, Bayerwerk

Zentralbereich
Patente, Marken und Lizenzen  Bg-by-c

Verfahren zur Herstellung von Tetramethylencarbonat und Bis-tetramethylendicarbonat
_____

Die Erfindung betrifft ein Verfahren zur Herstellung von Tetramethylencarbonat und Bis-tetramethylendicarbonat sowie den neuen cyclischen Kohlensäureester, das Tetramethylencarbonat. Weiterhin betrifft die Erfindung die Verwendung des Tetramethylencarbonats und des Bis-tetramethylendicarbonats zur Herstellung von hochmolekularen Polycarbonaten.

Es ist bekannt, daß die Herstellung von cyclischen Kohlensäureestern von Butandiol-1,4 durch thermische Depolymerisation des entsprechenden Polycarbonats nur äußerst schwierig zu bewerkstelligen ist, da dabei im überwiegendem Maße Tetrahydrofuran gebildet wird. So ist zum Beispiel aus J. Amer. Soc. Chem. 52, 314 (1930) bekannt, daß bei der Depolymerisation von Tetramethylenpolycarbonat bei 300°C unter vermindertem Druck das Bis-tetramethylendicarbonat nur in einer Ausbeute von weniger als 1 % erhalten wird.

Es wurde nun gefunden, daß man cyclische Kohlensäureester aus Butandiol-1,4, wie Tetramethylencarbonat und Bis-tetramethylendicarbonat, in technisch interessanten Ausbeuten herstellen kann, wobei die Bildung des Tetra-

Le A 21 539-Ausland

- 2 -

hydrofurans weitgehend unterdrückt wird, wenn man die Depolymerisation mit einem sorgfältig gereinigten, von Katalysatoren, deren Folgeprodukte und/oder sonstigen Fremdstoffen befreiten Tetramethylenpolycarbonat durchführt.

Gegenstand der vorliegenden Erfindung ist daher ein Verfahren zur Herstellung von Tetramethylencarbonat und Bis-tetramethylendicarbonat durch Depolymerisation von Tetramethylenpolycarbonat, das dadurch gekennzeichnet ist, daß man aus dem Tetramethylenpolycarbonat die zu dessen Herstellung verwendeten Katalysatoren, deren Folgeprodukte und/oder sonstigen Fremdstoffe entfernt, das so vorbehandelte Polycarbonat auf zur Depolymerisation ausreichende Temperatur erhitzt und die entstehenden cyclischen Kohlensäureester durch Destillation unter vermindertem Druck abtrennt.

Ein weiterer Gegenstand der vorliegenden Erfindung ist der neue cyclische Kohlensäureester, das Tetramethylencarbonat.

Das als Ausgangsmaterial dienende Tetramethylenpolycarbonat kann in bekannter Weise hergestellt werden, zum Beispiel durch Umesterung von Butandiol-1,4 mit Diethylcarbonat unter katalytischer Wirkung von Alkali, wobei darauf zu achten ist, daß die Umesterungstemperaturen möglichst niedrig bleiben ( $< 130°$), um Verluste durch Decarboxylierung zu vermeiden.

Le A 21 539

- 3 -

Das erfindungsgemäß einzusetzende Tetramethylenpolycarbonat kann beispielsweise Molekulargewichte im Bereich
von 5000 bis 50 000 aufweisen.

Es ist ein wesentliches Merkmal des erfindungsgemäßen
Verfahrens, daß vor der Depolymerisation des Tetramethylenpolycarbonats aus dem Polycarbonat die zu seiner Herstellung
verwendeten Katalysatoren, deren Folgeprodukte und/oder
sonstige im Polycarbonat vorhandene Fremdstoffe entfernt
werden. Die Entfernung dieser Stoffe kann auf verschiedene Weise vorgenommen werden.

Vorzugsweise löst man dazu das Polycarbonat zunächst in
einem geeigneten Lösungsmittel, z.B. in Methylenchlorid
oder Toluol. Die so erhaltene Polycarbonatlösung kann
dann beispielsweise mit Wasser, insbesondere mit destilliertem oder demineralisiertem Wasser, gewaschen werden,
beispielsweise 2- bis 5-mal. Vorzugsweise behandelt man
dabei die Polycarbonatlösung vor der Wasserwäsche mit
einer verdünnten Säure, z.B. mit bis zu 20 gew.-%iger
wäßriger Salzsäure oder Schwefelsäure. Die Entfernung
der zur Herstellung des Polycarbonats verwendeten Katalysatoren, deren Folgeprodukte und/oder sonstiger vorhandener Fremdstoffe kann auch durchgeführt werden, indem
man die Polycarbonatlösung mit einem Ionenaustauscher behandelt. Vorzugsweise werden hierzu säureaktivierte
Bleicherden und/oder sulfonierte vernetzte Polystyrole
eingesetzt.

Im allgemeinen ist es besonders vorteilhaft, die

Le A 21 539

- 4 -

bei der Herstellung des Polycarbonats verwendeten Katalysatoren, deren Folgeprodukte und sonstiger
vorhandener Fremdstoffe (z.B. Wasser, Säuren, Lösungsmittel und/oder Ionenaustauscher) vor der Depolymerisation
möglichst vollständig zu entfernen.

Die Depolymerisation des so vorbehandelten Tetramethylenpolycarbonats erfolgt dann durch Erhitzen. Hierzu sind
im allgemeinen Temperaturen von ca. 200 bis 300°C erforderlich. Vorzugsweise arbeitet man bei Temperaturen von
220 bis 260°C.

Gemäß einer anderen Variante des erfindungsgemäßen Verfahrens kann man die Depolymerisation des Tetramethylenpolycarbonats auch in Gegenwart von hochsiedenden, inerten,
organischen Kohlenwasserstoffen und/oder in Gegenwart eines
inerten Gases durchführen. Dadurch ist es möglich, das
Verhältnis der beiden cyclischen Kohlensäureester zu
Gunsten des Tetramethylencarbonats derart zu verschieben,
daß dies zum Hauptprodukt wird.

Als inerte Gase kommen z.B. Stickstoff, Kohlenstoffdioxid oder Edelgase in Frage.

Als hochsiedende, inerte, organische Kohlenwasserstoffe
eignen sich beispielsweise gesättigte Kohlenwasserstoffe
mit Siedepunkten, die bei etwa 150 bis 450°C liegen,
wie Cumol, Cymol, Diphenylpropan, Naphthalin, Decahydronaphthalin, Chlornaphthaline, Terphenyle und Mehrkernkohlenwasserstoffe auf der Basis von Diisopropylbenzol,

Le A 21 539

- 5 -

wie sie in Makromol. Chem. 177, 3515 (1976) beschrieben sind.

Die während der Depolymerisation entstehenden Kohlensäureester werden vorzugsweise in dem Maße entfernt, wie sie sich bilden. Dies geschieht dadurch, daß man die Depolymerisation im Vakuum durchführt. Das Vakuum wird dabei zweckmäßigerweise so gewählt, daß die gebildeten Kohlensäureester abdestillieren und nach Kühlung in einer Vorlage aufgefangen werden können. Man arbeitet beispielsweise bei Drucken im Bereich von etwa 0,001 bis 100 mbar, vorzugsweise bei 0,01 bis 25 mbar.

Die bei der Depolymerisation erhaltenen cyclischen Kohlensäureester können, falls dies gewünscht wird, weiter gereinigt werden. Diese Reinigung kann beispielsweise durch Umkristallisation aus einem geeigneten Lösungsmittel (z.B. Essigsäureethylester, Toluol, Cyclohexan oder Tetrachlorkohlenstoff) oder durch fraktionierte Destillation im Vakuum erfolgen.

Auf diese Weise erhält man den neuen cyclischen Kohlensäureester, das Tetramethylencarbonat.

Das erfindungsgemäß hergestellte Tetramethylencarbonat und das Bis-tetramethylendicarbonat besitzen technisches Interesse als Polymerisationskomponenten. Sie können so für die Herstellung von hochmolekularen Polycarbonaten verwendet werden. Zum Beispiel kann man durch Polymerisation von Tetramethylencarbonat und Bis-tetramethylen-

Le A 21 539

dicarbonat Homopolymerisate mit hohen Molekulargewichten (bis zu 100 000) herstellen, ohne Gefahr zu laufen, daß die bei hohen Temperaturen, wie sie bei der Herstellung von Polycarbonaten durch Polykondensation unvermeidlich sind, üblicherweise beobachteten Nebenreaktionen, wie Decarboxylierung und Kettenabbruch, eintreten und zu einer Qualitätsverminderung des Homopolymerisats führen.

Durch Copolymerisation des Tetramethylencarbonats und des Bis-tetramethylendicarbonats mit anderen Ringcarbonaten, wie Trimethylencarbonat, Neopentylglykolcarbonat oder Bishexamethylendicarbonat, können erwünschte Abwandlungen der Polycarbonat-Eigenschaften, wie höhere Elastizität, größere Steifigkeit oder Unterdrückung der Kristallisationstendenz, erzielt werden.

Diese Variationsmöglichkeiten können für die Herstellung von vernetzten, unschmelzbaren und unlöslichen Polycarbonaten durch Copolymerisation mit bifunktionellen Ringcarbonaten, wie Ditrimethylolpropan-dicarbonat, Pentaerythritdicarbonat und Di-(trimethylolpropancarbonat)-carbonat, von großer Bedeutung sein.

Durch Copolymerisation von Tetramethylencarbonat und Bis-tetramethylendicarbonat mit Lactamen, wie Caprolactam, werden besonders interessante Copolymerisate erhalten, die als Klebstoffe Verwendung finden können.

Das Tetramethylencarbonat ist ein besonders reaktionsfähiger cyclischer Kohlensäureester, der sich im Gegensatz zu den bekannten Ringcarbonaten, wie Trimethylen-

Le A 21 539

carbonat, 3,3-Dimethyl-trimethylencarbonat oder Ethylencarbonat, leicht rein thermisch in Abwesenheit von Katalysatoren zu hochmolekularem Tetramethylenpolycarbonat polymerisieren läßt.

Le A 21 539

- 8 -

Beispiel 1

450 g (5 Mol) Butandiol-1,4, 650 g (5,5 Mol) Diethylencarbonat und 1 g Kaliumcarbonat werden an einer 1,2-m-Füllkörperkolonne auf 110-130°C Innentemperatur erhitzt, während im Laufe von 1 1/2 h 320 g Ethanol über Kopf bei 78°C abdestillieren. Den Rest des Alkohols (140 g) entfernt man durch allmähliche Verminderung des Drucks auf 25 mbar bei unveränderter Innentemperatur. Man löst das Reaktionsprodukt in Methylenchlorid und läßt es zur Entfernung des Katalysators über einen Ionentauscher (sulfoniertes vernetztes Polystyrol) laufen. Man entfernt das Lösungsmittel bei Normaldruck und erhitzt den Rückstand auf 150°C unter 300-314 mbar und schließlich bis 175°C/0,4 mbar. Man erhält 585 g Tetramethylenpolycarbonat, eine bei Raumtemperatur opake, farblose Masse.

60 g des Tetramethylenpolycarbonats werden bei vermindertem Druck in einem Säbelkolben unter Rühren auf 220 bis 240°C/0,03 mbar erhitzt, während im Laufe von 5 1/2 Stunden ein teilweise erstarrendes Destillat in die Vorlage geht. Flüchtige Bestandteile, Tetrahydrofuran und Kohlendioxid werden in 2 Kühlfallen, beschickt mit Trockeneis bzw. flüssigem Stickstoff, aufgefangen. Ein bei 150-160°C gehaltenes Heizband verhindert das Ankrusten von Kristallen im aufsteigenden Rohr und im Übergang. Das Destillat (49,5 g) wird mit Essigester ausgewaschen. 27,5 g Kristalle, nahezu reines Bis-tetramethylendicarbonat vom Schmp. 170-174°C bleiben ungelöst. Ausbeute

- 9 -

46 % d. Th. Die Mutterlauge wird eingeengt und aus Toluol-Cyclohexan umkristallisiert. Man erhält 15,9 g einer Verbindung vom Schmp. 77-78°C, des monomeren Tetramethylencarbonats. Ausbeute 27 % d. Th., Sdp. 113°C/13 mbar.

$C_5H_8O_3$ (116.1) Ber. C 51,72   H 6,95   O 41,34
              Gef.   51,65      6,99      41,29

Das NMR-Spektrum ist in Übereinstimmung mit dem monomeren Tetramethylencarbonat. Es macht 38 % der Gesamtausbeute aus.

Beispiel 2

In einen auf 240-260°C erhitzten Claisen-Kolben läßt man unter vermindertem Druck die Schmelze von 100 g Tetramethylenpolycarbonat und 50 g 1,4-Bis-(2-phenyl-2-propyl)-benzol innerhalb von 6 1/2 h durch einen auf 160°C erhitzten Tropftrichter eintropfen, während ein teilweise kristallisiertes Destillat bei 150-200°C/0,03 mbar übergeht: 117 g. Das Destillat wird mit der dreifachen Menge Essigester verrührt und abgesaugt. Man erhält 22 g Bis-tetramethylendicarbonat vom Schmp. 168-172°C. Die Mutterlauge wird eingeengt und in 80 ml Toluol gelöst. Man versetzt mit 220 ml Cyclohexan und erhält durch Abkühlen 30 g Tetramethylencarbonat vom Schmp. 74-76°C. Gesamtausbeute 52 % d. Th. Das Monomere macht 58 % der Gesamtcarbonatmenge aus.

Le A 21 539

- 10 -

Beispiel 3

40 g Tetramethylenpolycarbonat und 40 g 1,4-Bis-$\underline{/}$2-(4-methylphenyl)-2-propyl$\underline{/}$-benzol werden in einem Claisen-Kolben auf 225-240°C/1 mbar erhitzt, während durch eine Kapillare Kohlendioxid durch die Schmelze geleitet wird. Im Laufe von 5 h werden in den mit weiten Abgängen versehenen zwei mit Eiswasser gekühlten Vorlagen 30 g vorwiegend kristallines Destillat aufgefangen.

In der mit flüssigem Stickstoff gefüllten Kühlfalle ist Kohlendioxid und eine kleine Menge Tetrahydrofuran kondensiert. Durch Redestillation des Reaktionsproduktes erhält man 12,5 g Tetramethylencarbonat vom Sdp. 110 bis 112°C/13 mbar und 3 g Bis-tetramethylen-dicarbonat vom Sdp. 170-180°C/13 mbar. Der Rückstand ist der als Schleppmittel benutzte Dreikernkohlenwasserstoff. Der Gehalt an monomerem Carbonat beträgt 80 % der Gesamtausbeute.

Beispiel 4

40 g Bis-tetramethylen-dicarbonat und 4 mg Thalliumisooctoat werden unter Rühren und Überleiten von trockenem Stickstoff auf 180°C erhitzt. Nach 40 Min. wird die Schmelze zäh und nach 1 1/2 h Reaktionszeit ist die Polymerisation beendet. Man erhält ein bei Raumtemperatur opakes, zähes farbloses Polymerisat. Es läßt sich bei Raumtemperatur zu Fäden von beträchtlicher Reißfestig-

Le A 21 539

keit verstrecken. Das Schmelzintervall liegt bei 70-80°C. Das Molekulargewicht, bestimmt durch Lichtzerstreuung, beträgt 28 500.

Beispiel 5

23,2 g Tetramethylencarbonat, 22 g Caprolactam und 0,7 g einer dreiprozentigen Lösung von Natrium in Caprolactam werden unter Rühren und Überleiten von trockenem Stickstoff auf 160°C erhitzt. Nach einer Stunde wird die Schmelze zähflüssig. Nach 3 h Reaktionszeit erhält man ein bei Raumtemperatur opakes, gelbstichiges, gummiartiges, zähes Polymerisat. Es kann bei Raumtemperatur zu reißfesten Fäden verstreckt werden. Der Schmelzbereich liegt bei 55-63°C. Das Molekulargewicht, gemessen durch Lichtzerstreuung, beträgt 22 700.

Beispiel 6

25 g Tetramethylencarbonat und 2,5 g Ditrimethylolpropan-dicarbonat werden auf 140°C erhitzt. Nach 2 h ist die Schmelze geliert. Man erhält nach Abkühlen ein unschmelzbares und in Lösungsmittel unlösliches, farbloses, opakes Copolymerisat von beträchtlicher Zähigkeit und Steifigkeit.

Beispiel 7

23,2 g Tetramethylencarbonat und 26 g Neopentylglykolcarbonat werden auf 140°C erhitzt. Nach 70 Min. ist die

Le A 21 539

Schmelze so zäh geworden, daß sie nicht mehr fließt. Man erhält nach Abkühlen ein gummiartiges, plastisches, farbloses und transparentes Copolymerisat.

- 13 -

Patentansprüche

1. Verfahren zur Herstellung von Tetramethylencarbonat und Bis-tetramethylendicarbonat durch Depolymerisation von Tetramethylenpolycarbonat, dadurch gekennzeichnet, daß man aus dem Tetramethylenpolycarbonat die zu dessen Herstellung verwendeten Katalysatoren, deren Folgeprodukte und/oder sonstigen Fremdstoffe entfernt, das so vorbehandelte Polycarbonat auf zur Depolymerisation ausreichende Temperatur erhitzt und die entstehenden cyclischen Kohlensäureester durch Destillation unter vermindertem Druck abtrennt.

2. Verfahren nach Anspruch 1, dadurch gekennzeichnet, daß man die Depolymerisation bei Temperaturen im Bereich von 200 bis 300°C durchführt.

3. Verfahren nach Anspruch 1, dadurch gekennzeichnet, daß man die Depolymerisation bei Temperaturen im Bereich von 220 bis 260°C durchführt.

4. Verfahren nach Ansprüchen 1 bis 3, dadurch gekennzeichnet, daß man die Depolymerisation bei Drucken im Bereich von 0,001 bis 100 mbar durchführt.

5. Verfahren nach Ansprüchen 1 bis 4, dadurch gekennzeichnet, daß man die Depolymerisation in Gegenwart von hochsiedenden, inerten organischen Kohlenwasserstoffen und/oder in Gegenwart eines inerten Gases durchführt.

Le A 21 539

- 14 -

6. Tetramethylencarbonat

7. Verwendung von Tetramethylencarbonat und Bis-tetramethylendicarbonat als Polymerisationskomponenten für die Herstellung hochmolekularer Poly-carbonate.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,X | JOURNAL OF AMERICAN CHEMICAL SOCIETY, Band 52, Januar 1930, Seiten 314-326, Washington, USA W.H. CAROTHERS et al.: "Studies on polymerization and ring formation. III. Glycol esters of carbonic acid" * Seiten 314-316, Tabelle I; Seite 321-324 Zusammenfassung * | 1-4,6, 7 | C 07 D 321/06 C 08 J 11/00 C 07 D 323/00 |
| | --- | | |
| A | JOURNAL OF AMERICAN CHEMICAL SOCIETY, Band 55, Dezember 1933, Seiten 5031-5039, Washington, USA J.W. HILL et al.: "Studies of polymerization and ring formation. XX. Many-membered cyclic esters" * Seiten 5031-5032, Tabelle I; Seite 5035 * | 1-4,6, 7 | |
| | | | **RECHERCHIERTE SACHGEBIETE (Int. Cl. 3)** |
| A | EP-A-0 000 904 (BAYER) * Insgesamt * | 1-3 | C 07 D 321/00 C 08 J 11/00 C 07 D 323/00 |
| | ----- | | |

Der vorliegende Recherchenbericht wurde für alle Patentansprüche erstellt.

| Recherchenort DEN HAAG | Abschlußdatum der Recherche 17-05-1983 | Prüfer FRANCOIS J.C.L. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82